# EUROPÄISCHE PATENTSCHRIFT

(11) **EP 1 474 440 B1**
(45) Veröffentlichungstag und Bekanntmachung des Hinweises auf die Patenterteilung: **10.09.2008**
(21) Anmeldenummer: 02777014.8
(22) Anmeldetag: 06.09.2002
(51) Int. Cl.: C07K 5/06, C07K 5/083, A61K 38/05, A61K 38/06, A61K 31/4015, A61P 25/28

(54) **CONIOSULFIDE UND DEREN DERIVATE, VERFAHREN ZUR IHRER HERSTELLUNG UND VERWENDUNG ALS ARZNEIMITTEL**
CONIOSULPHIDES AND THEIR DERIVATIVES, METHOD FOR THEIR PRODUCTION AND USE AS MEDICAMENTS
CONIOSULFURES ET DERIVES, LEURS PROCEDES DE PRODUCTION ET LEUR UTILISATION EN TANT QUE MEDICAMENTS

(30) Priorität: 22.09.2001 DE 10146737
(43) Veröffentlichungstag der Anmeldung: 10.11.2004
(60) Teilanmeldung: 08000437.7
(73) Patentinhaber: Sanofi-Aventis Deutschland GmbH, 65929 Frankfurt am Main (DE)
(72) Erfinder: VERTESY, László, 65817 Eppstein-Vockenhausen (DE); EHRLICH, Klaus, 65428 Rüsselsheim (DE); KURZ, Michael, 65719 Hofheim (DE); SEGETH, Marian, Paul, 65510 Idstein (DE); TOTI, Luigi, 65239 Hochheim (DE)
(86) Internationale Anmeldenummer: PCT/EP2002/009977
(87) Internationale Veröffentlichungsnummer: WO 2003/029495

(56) Entgegenhaltungen:
- WO-A-01/59104
- WO-A-98/21327
- FR-A- 2 740 454
- US-A- 5 420 155
- CUTLER N R ET AL: "Review of the next generation of Alzheimer's disease therapeutics: challenges for drug development." PROGRESS IN NEURO-PSYCHOPHARMACOLOGY & BIOLOGICAL PSYCHIATRY. ENGLAND JAN 2001, Bd. 25, Nr. 1, Januar 2001 (2001-01), Seiten 27-57, XP002243957 ISSN: 0278-5846
- FASSBENDER K ET AL: "Alzheimer's disease: molecular concepts and therapeutic targets." DIE NATURWISSENSCHAFTEN. GERMANY JUN 2001, Bd. 88, Nr. 6, Juni 2001 (2001-06), Seiten 261-267, XP002243958 ISSN: 0028-1042

## Beschreibung

Coniosulfide und deren Derivate, Verfahren zur ihrer Herstellung und Verwendung als Arzneimittel.

Die Alzheimer'sche Krankheit (Demenz vom Alzheimer-Typ, AD) ist nach heutigem Kenntnisstand eine erbliche, unaufhaltsam fortschreitende Großhirnrinden-Atrophie mit zunehmenden Denkstörungen. Bei der Alzheimer-Krankheit handelt es sich um eine neuropsychiatrische Erkrankung, die vorwiegend bei älteren Menschen auftritt. Die Erkrankung manifestiert sich in einem Symptomenkomplex, die Gedächtnisstörungen, herabgesetzte Merkfähigkeit, Orientierungsstörungen, Sprachstörungen, Störungen des gezielten Denken usw. einschließt. Bei Alzheimer-Patienten findet man charakteristische neurohistologische Veränderungen, wie beispielsweise die Ablagerungen sogenannter amyloider Plaques sowie auch eine Degeneration der Neurofibrillen in den Nervenzellen (sog. "fibrilläre Bündel"). Diese Veränderungen sind zwar charakteristisch, jedoch keineswegs spezifisch, da sie in geringerem Ausmaß auch beim normalen Alterungsprozess auftreten.

Derzeit ist keine kausale, sondern nur eine symptomatische Behandlung der AD möglich. Bisher gibt es Medikamente, die den Verlauf der Krankheit lediglich verzögern, sie aber nicht heilen können. Den wichtigsten therapeutischen Ansatz bietet die Gruppe der cerebralen Acetylcholinesterase-Hemmer (Tacrin^{®}, Donepezil^{®}, Rivastigmin^{®}, Galantamin^{®}), da für die gedächtnisrelevanten Strukturen, die bei AD in besonderem Maße beeinträchtigt sind, die cholinerge Signalübertragung eine große Rolle spielt. Die Medikamente können allerdings nur bei leichtem und mittlerem Krankheitsstadium eingesetzt werden. Sie erhöhen die Konzentration von Acetylcholin in den informationsübertragenden Synapsen des Gehirns. Bei zu starker Schädigung der Neuronen, also im Spätstadium der Krankheit, sind sie nicht mehr wirksam. Weitere Substanzen, deren Einsatz überprüft wird, sind Östrogene, nicht-steroidale Analgetika, Antioxidantien und Nerven-Wachstumsfaktoren (NGF). Alle diese Mittel sind aber in ihrer Wirkung zur Behandlung der Alzheimer'schen Erkrankung unzulänglich.

Man schätzt, dass es gegenwärtig etwa eine Million Menschen in der Bundesrepublik Deutschland gibt, die an der Alzheimer'schen Krankheit leiden. Diese Zahl wird vermutlich aufgrund der steigenden Lebenserwartung der Bevölkerung in den nächsten Jahren noch weiter ansteigen (F. Kohl, Prax. Naturwiss. Biol. (1999), 48(6), 26-31). Daher sind dringend neue Substanzen zur Behandlung dieser Erkrankung notwendig.

Die für die AD charakteristischen histologischen Gehirnveränderungen sind die sog. Amyloid-Plaques. Es sind krankhafte Proteinablagerungen des ß-Amyloid-Peptids bzw. des ßA4-Proteins, welches, infolge eines Stoffwechseldefektes, aus einem physiologischen Zellmembran-Bestandteil, dem Amyloid Precursor Protein (APP), heraus gespalten wird. Es reichert sich dann innerhalb des Gehirns an, wo es vom Körper nicht mehr abgebaut werden kann und als Plaque erscheint (F. Kohl, Prax. Naturwiss. Biol. (1999), 48(6), 26-31; D. J. Selkoe; Trends Cell Biol. (1998), 8, 447-453). Das wesentliche Element des Amyloid-Plaques ist ein 39 bis 43 Aminosäuren umfassendes Peptid, das sogenannte β-Amyloid oder Aß. Dieses Peptid entsteht bei der Prozessierung des APP. Bei Patienten, die unter der erblichen Form der AD leiden, sind Mutationen in den Genen, die für dieses Prozessieren kodieren, gefunden worden. In denselben Fällen wurde eine Zunahme der Aß-Produktion beobachtet, woraus geschlossen worden ist, daß das Prozessieren des APP ein geeigneter Angriffsort für die Behandlung der Alzheimer'schen Erkrankung sein könnte. Beim Prozessieren des APP spielt die Tetrapeptid-Teilsequenz Asn-Pro-Thr-Tyr eine Rolle. Diese Teilsequenz wird von den Proteinen COFE65 und besonders FE65 erkannt und es wird angenommen, daß die für die APP-Prozessierung verantwortliche Protein-Protein-Wechselwirkung mit ihrer Beteiligung stattfindet (WO 98/21327). Agentien, die die APP - FE65 - Wechselwirkung hemmen, sollten daher wertvolle Mittel zur Behandlung von AD sein.

In FR2740454 werden Polypetide beschrieben, die die Hemmung der Wechselwirkung von FE65 mit APP bewirken und damit zur Bekämpfung der Alzheimer'schen Krankheit eingesetzt werden können.

Die vorliegende Erfindung betrifft neue Wirkstoffe (Coniosulfide) vom FarnesylthioPeptid-Typ, die von dem Mikroorganismus *Coniochaeta ellipsoidea* Udagawa, DSM 13856, während der Fermentation gebildet werden, von den Coniosulfiden abgeleitete chemische Derivate, ein Verfahren zu deren Herstellung, und die Verwendung von Coniosulfiden und ihren Derivaten als Arzneimittel.

Es sind bereits einige Verbindungen mit Farnesylthiopeptid-Grundstruktur bekannt.

Eng Wui Tan et al. (J. Am. Chem. Soc. (1991), 113, 6299-6300) und Bryant A. Gilbert (J. Am. Chem. Soc. (1992), 114, 3966-3973) beschreiben die Verbindungen Farnesylcystein und Farnesylcysteinoxid, 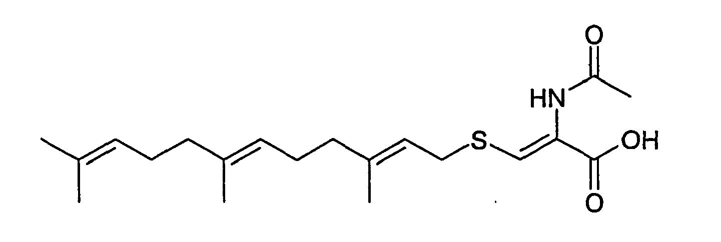 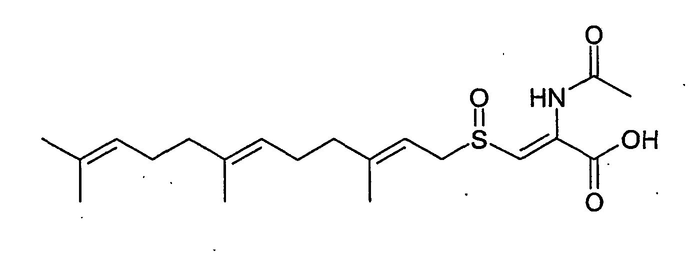 die gute Substrate der isoprenylierten Proteinmethyltransferase sind.

Zuvor war schon die Bedeutung der Prenylierung von Proteinen für deren Verankerung in biologische Membranen bekannt (J. A. Glomset et al., Trends in Biochem. Sciences (1990), 15, 139-142).

Miyakawa et al. (J. Bacteriol. (1982), 151, 1184-1194) beschreiben die Verbindungen Rhodotorucine A (p=0) und Rhodotorucine-A-S-oxid (p=1), 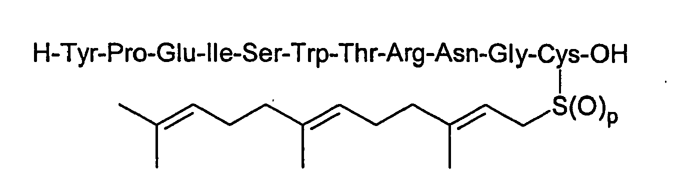 als *Rhodosporidium toruloides* Typ-A-Zell Sexualhormone.

Es wurde überraschend gefunden, daß der Stamm *Coniochaeta ellipsoidea* Udagawa, DSM 13856, neue Verbindungen zu bilden vermag, welche nicht nur die APP-FE65-Wechselwirkung wirksam hemmen, sondern auch gut verträglich sind. Diese Verbindungen sind die nachfolgend beschriebenen Coniosulfide genannten Famesylthiopeptid-Derivate der Formel (I).

Gegenstand der Erfindung ist eine Verbindung der Formel (I) 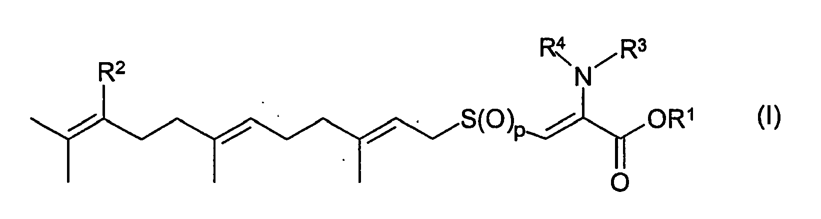 wobei
- R¹: 1.0 H, oder
2.0 einen Rest ausgewählt aus -C₁-C₆-Alkyl, -C₂-C₆-Alkenyl, -C₂-C₆-Alkinyl oder -C₆-C₁₀-Aryl, der ein- oder zweifach substituiert sein kann durch
2.1 -OH,
2.2 =O,
2.3 -O-C₁-C₆-Alkyl,
2.4 -O-C₂-C₆-Alkenyl,
2.5 -C₆-C₁₀-Aryl,
2.6 -NH-C₁-C₆-Alkyl,
2.7 -NH-C₂-C₆-Alkenyl,
2.8 -NH₂ oder
2.9 Fluor, Chlor, Brom oder lod,
   worin die Substituenten 2.3 bis 2.8 auch noch weiter substituiert sein können durch -CN oder -Amid-Funktionen,

3.0 -(C₁-C₄-Alkyl)-(C₆-C₁₀-Aryl), oder
4.0 -NH₂, -NH-(C₁-C₆-Alkyl), -NH-(C₂-C₆-Alkenyl) ist,
- R²: H, Methyl, Ethyl, n-Propyl, iso-Propyl, n-Butyl oder iso-Butyl,
- R³: einen aus 1 bis 10 Aminosäuren bestehenden Rest, der N-terminal geschützt sein kann mit einer -CO-(C₁-C₆-Alkyl)- oder einer -C₁-C₆-Alkyl-Gruppe,
- R⁴: H, -C₁-C₆-Alkyl, -CO-(C₁-C₆-Alkyl), -CO-(C₂-C₆-Alkenyl) oder -(C₁-C₄-Alkyl)-(C₆-C₁₀-Aryl), und
- p: eine ganze Zahl 0, 1 oder 2 bedeutet,
ausgenommen Verbindungen der Formel (I), in denen gleichzeitig
- R¹: H,
- R²: H,
- R³: -CO-CH₃,
- R⁴: H, und
- p: 0 oder 1 bedeuten,
und/oder eine stereoisomere Form der Verbindung der Formel (I) und/oder Gemische dieser Formen in jedem Verhältnis,
und/oder Komplexe oder Addukte der Verbindung der Formel (I),
und/oder ein physiologisch verträgliches Salz der Verbindung der Formel (I).

R¹ ist vorzugsweise H.

R² ist vorzugsweise H oder Methyl, besonders bevorzugt Methyl.

R³ ist vorzugsweise ein aus 1 bis 2 Aminosäuren, bevorzugt natürlichen Aminosäuren, besonders bevorzugt ungeladenen natürlichen Aminosäuren, bestehender Rest, der N-terminal geschützt sein kann, vorzugsweise mit einer Acetylgruppe.

R⁴ ist vorzugsweise H.

p ist vorzugsweise 0.

Die Erfindung betrifft vorzugsweise eine Verbindung der Formel (I), in der R⁴ H bedeutet, und R¹, R², R³ und p wie oben definiert sind.

Ferner betrifft die Erfindung bevorzugt eine Verbindung der Formel (I), in der R⁴ Wasserstoff und p Null bedeutet, und R¹, R² und R³ wie oben definiert sind.

Besonders bevorzugt betrifft die Erfindung eine Verbindung der Formel (I), in der R² H oder Methyl ist, R⁴ Wasserstoff, und p Null bedeutet, und R¹ und R³ wie oben definiert sind.

Ferner betrifft die Erfindung besonders bevorzugt eine Verbindung der Formel (I), in der R² H oder Methyl ist, R³ einen aus 1 bis 2 Aminosäuren bestehenden Rest, der N-terminal geschützt sein kann, R⁴ Wasserstoff, und p Null bedeutet, und R¹ wie oben definiert sind.

Speziell bevorzugt betrifft die Erfindung eine Verbindung der Formel (I), in der R¹ H, R² H oder Methyl, R³ ein aus 1 bis 2 Aminosäuren bestehender Rest, der N-terminal geschützt sein kann, R⁴ Wasserstoff, und p Null bedeutet.

Besonders speziell bevorzugt betrifft die Erfindung eine Verbindung der Formel (I), in der R¹ Wasserstoff, R² Wasserstoff oder Methyl, R³ ein Rest Gly oder Gly-Gly, der N-terminal geschützt sein kann, R⁴ Wasserstoff, und p Null bedeutet.

Aminosäuren können D- oder L-Konfiguration besitzen. Natürliche Aminosäuren sind die kodierbaren Aminosäuren und besitzen L-Konfiguration. Natürliche neutrale Aminosäuren sind Glycin (Gly), L-Alanin (Ala), L-Valin (Val), L-Leucin (Leu), L-Isoleucin (IIe), L-Prolin (Pro), L-Tryptophan (Typ), L-Phenylalanin (Phe) und L-Methionin(Met).

C₁-C₆-Alkyl bedeutet eine geradkettige oder verzweigte Alkylgruppe mit 1 bis 6 C-Atomen, bevorzugt mit 1 bis 4 C-Atomen, beispielsweise Methyl, Ethyl, n-Propyl, i-Propyl, n-Butyl, tert.Butyl oder n-Hexyl.

C₂-C₆-Alkenyl bedeutet eine geradkettige oder verzweigte Alkenylgruppe mit 2 bis 6 C-Atomen, die einfach, zweifach oder dreifach ungesättigt ist, wie z.B. Allyl, Crotyl, 1-Propenyl, Penta-1,3-dienyl oder Pentenyl.

C₂-C₆-Alkinyl bedeutet eine geradkettige oder verzweigte Alkinylgruppe mit 2 bis 6 C-Atomen, die eine oder zwei Dreifachbindungen enthält, beispielsweise Propinyl, Butinyl oder Pentinyl.

C₆-C₁₀-Aryl bedeutet eine Arylgruppe mit 6 bis 10 Ring-C-Atomen, zum Beispiel Phenyl oder Naphthyl, die einfach oder mehrfach substituiert sein kann mit beispielsweise Chlor, Brom, Fluor, lod,- C₁-C₄-Alkyl, vorzugsweise Methyl, Hydroxy,-O-(C₁-C₄-Alkyl), vorzugsweise Methoxy, oder durch perfluorierte C₁-C₄-Alkylreste, vorzugsweise Trifluormethyl.

-(C₁-C₄-Alkyl)-(C₆-C₁₀-Aryl) bedeutet vorzugsweise Benzyl.

-CO-(C₁-C₆-Alkyl) bedeutet eine aliphatische Acylgruppe mit 2 bis 7 C-Atomen, vorzugsweise 2 bis 5 C-Atomen, zum Beispiel Formyl, Acetyl, Propionyl, Butyryl, Hexanoyl, Acryloyl, Crotonoyl, Propioloyl, die noch weiter substituiert sein kann, beispielsweise durch Chlor, Brom, Fluor, lod, NH₂, -(C₁-C₄-Alkyl)-NH₂, vorzugsweise Methylamino oder Ethylamino.

-CO-(C₂-C₆-Alkenyl) bedeutet eine aliphatische Acylgruppe mit 3 bis 7 C-Atomen, vorzugsweise 3 bis 5 C-Atomen, zum Acryloyl, Crotonoyl, Propioloyl, die noch weiter substituiert sein kann, beispielsweise durch Chlor, Brom, Fluor, lod, NH₂, -(C₁-C₄-Alkyl)-NH₂, vorzugsweise Methylamino oder Ethylamino.

-CO-(C₆-C₁₀-Aryl) bedeutet eine aromatische Acylgruppe mit 6 bis 10 Ring-C-Atomen, zum Beispiel Benzoyl oder Naphthoyl, das auch noch weiter substituiert sein kann, beispielsweise durch Chlor, Brom, Fluor, lod, C₁-C₄-Alkyl, vorzugsweise Methyl, Hydroxy, -(C₁-C₄-Alkyl)-NH₂ vorzugsweise Methylamino oder Ethylamino, oder -O-(C₁-C₇-Alkyl), vorzugsweise -O-(C₁-C₄-Alkyl), insbesondere Methoxy.

Chiralitätszentren in den Verbindungen der Formeln (I) können, wenn nicht anders angegeben, unabhängig voneinander in der R- oder in der S-Konfiguration vorliegen. Doppelbindungen können unabhängig voneinander in der cis- oder in trans-Position vorliegen. Die Erfindung betrifft sowohl die optisch reinen Verbindungen als auch Stereoisomeren-Gemische, wie Enantiomerengemische und Diasteromerengemische, in jedem Verhältnis.

Vorzugsweise betrifft die Erfindung eine Verbindung der Formel (II)

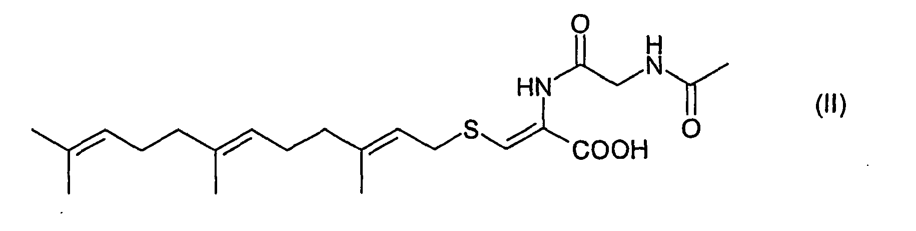

(Coniosulfid A: Summelformel: C₂₂H₃₄O₄N₂S, MG. 422.59),
eine Verbindung der Formel (III) 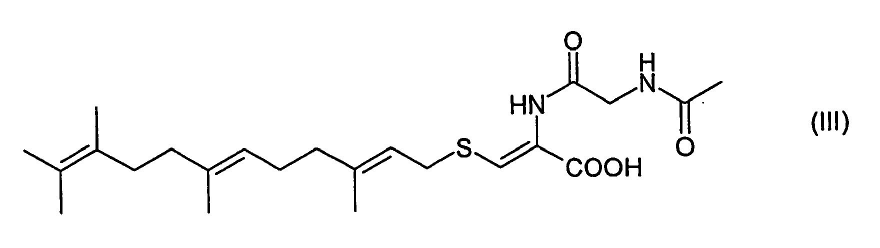 (Coniosulfid B, C₂₃H₃₆N₂O₄S, MG. 436.62),
eine Verbindung der Formel (IV) 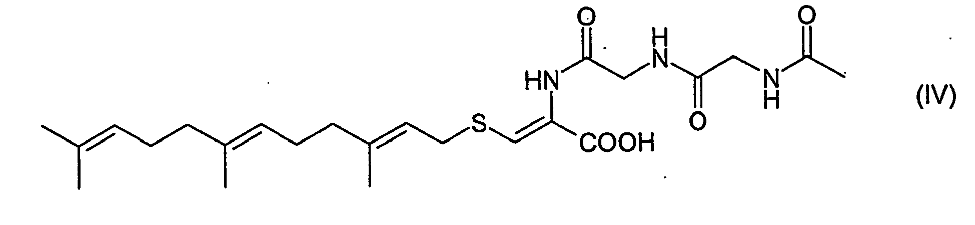 (Coniosulfid C, C₂₄H₃₇N₃O₅S, MG. 479.64) sowie
eine Verbindung der Formel (V) 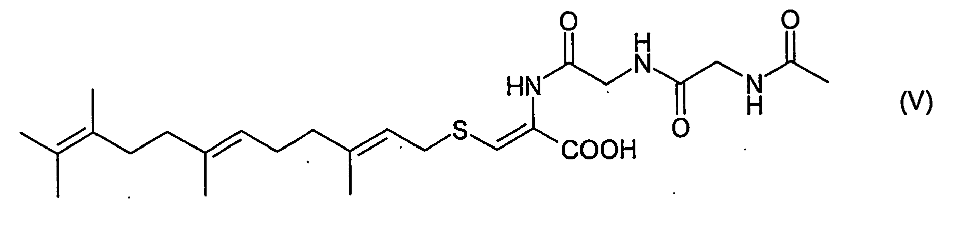 (Coniosulfid D, C₂₅H₃₉O₅N₃S, MG. 493.67),
sowie deren offensichtlichen chemischen Äquivalente und/oder physiologisch verträgliche Salze.

Offensichtliche chemische Äquivalente der Verbindungen der Formeln (I) bis (V) sind Verbindungen, die einen geringfügigen chemischen Unterschied aufweisen, also die gleiche Wirksamkeit haben oder sich unter milden Bedingungen in die erfindungsgemäßen Verbindungen umwandeln. Zu den genannten Äquivalenten gehören z.B. Ester, Azomethine (Schiff'sche Basen), Hydrierungsprodukte, Reduktionsprodukte, Komplexe oder Addukte der bzw. mit den erfindungsgemäßen Verbindungen, die nach in der Literatur bekannten Methoden hergestellt werden (J. March, Advanced Organic Synthesis, 4th Edition, John Wiley & Sons, 1992).

Doppelbindungen in der Alkylkette in Verbindungen der Formel (I) können beispielsweise durch Hydrogenolyse oder nach anderen an sich bekannte Methoden reduziert werden, zum Beispiel beschrieben von P. N. Rylander in "Hydrogenation Methods", Academic Press, New York (1985), Chapter 2, oder von H.O. House in "Modern Synthetic Reactions", W.A. Benjymin, Inc., New York (1972), Seite 446-452. Die Doppelbindungen können außerdem zu Epoxiden oxidiert werden, beispielsweise mittels meta-Chlorperbenzoesäure (MCPBA; J. March, Advanced Organic Synthesis, 4th Edition, John Wiley & Sons, 1992).

Die erfinderischen Verbindungen der Formel (I) bis (V) sowie die offensichtlichen chemischen Äquivalente derselben können nach dem Fachmann bekannten Methoden in die entsprechenden physiologisch verträglichen Salze übergeführt werden.

Unter physiologisch verträglichen Salzen der erfindungsgemäßen Verbindungen versteht man sowohl anorganische als auch organische Salze, wie sie in Remingtons Pharmaceutical Sciences (17. Auflage, Seite 1418 [1985]) beschrieben sind. Als Salze kommen insbesondere Alkali-, Ammonium-, Erdalkalisalze, Salze mit physiologisch verträglichen Aminen und Salze mit anorganischen oder organischen Säuren wie z.B. HCl, HBr, H₂SO₄, Maleinsäure, Fumarsäure in Frage.

Die erfindungsgemäßen Verbindungen der Formel (I) unterscheiden sich von literaturbekannten Substanzen. Es sind zwar strukturverwandte Farnesylthiopeptide beschrieben worden (vide supra), sie unterscheiden sich jedoch in ihrer exakten chemische Struktur und ihrer Wirksamkeit von den erfindungsgemäßen Verbindungen.

Die Erfindung betrifft außerdem eine Verbindung der Formel (I) erhältlich durch Fermentierung von *Coniochaeta ellipsoidea* Udagawa, DSM 13856, oder einer ihrer Mutanten oder Varianten in einem Kulturmedium, bis sich eine Verbindung der Formel (I) in dem Kulturmedium anhäuft, und anschließender Isolierung der Verbindung der Formel (I), und optional Derivatisierung und/oder gegebenenfalls Überführung in ein physiologisch verträgliches Salz.

Ferner betrifft die Erfindung ein Verfahren zur Herstellung einer Verbindung der Formel (I), dadurch gekennzeichnet, daß der Mikroorganismus *Coniochaeta ellipsoidea* Udagawa, DSM 13856, oder eine Variante oder Mutante von *Coniochaeta ellipsoidea* Udagawa, DSM 13856, in einem Kulturmedium fermentiert wird, bis sich eine Verbindung der Formel (I) in der Kulturbrühe anhäuft, die Verbindung der Formel (I) isoliert wird, und optional anschließend derivatisiert und gegebenfalls in ein pharmakologisch verträgliches Salz überführt wird.

Bei der Derivatisierung der Verbindung der Formel (I) wird nach an sich bekannten Methoden (J. March, Advanced Organic Chemistry, Wiley & Sons, 4th ed. 1992) beispielsweise die -NR³R⁴-Gruppe, in der R⁴ Wasserstoff bedeutet, alkyliert oder acyliert, das S-Atom der Sulfidgruppe (p=0) beispielsweise mittels MCPBA, Wasserstoffperoxid, Peressigsäure oder mittels anderer Persäuren zu einem Sulfoxid-Derivat (p=1) oder Sulfon-Derivat (p=2) oxidiert wird, und/oder eine freie Säuregruppe der Verbindung der Formel (I), in der R¹ Wasserstoff bedeutet, mit einem aktivierten Alkohol verestert. Aktivierte Alkohole sind zum Beispiel Diazomethan oder andere Alkohol-Derivate.

Um Umsetzungen selektiv durchzuführen, kann es vorteilhaft sein, in an sich bekannter Weise vor der Reaktion geeignete Schutzgruppen einzuführen. Die Schutzgruppen können nach der Reaktion abgespalten werden, und das Reaktionsprodukt wird anschließend gegebenenfalls gereinigt.

Der Pilz *Coniochaeta ellipsoidea* Udagawa wurde bei der Deutschen Sammlung von Mikroorganismen und Zellkulturen GmbH (DSM), Mascheroder Weg 1 B, 38124 Braunschweig, Deutschland nach den Regeln des Budapester Vertrages am 17.11.2000 unter der folgenden Nummer hinterlegt: DSM 13856. Der Pilz besitzt ein weisses Substratmycel und ganz wenig Luftmycel und bildet in Kultur keine für Coniochaeta charakteristischen Fruchtkörper.

Vorzugsweise umfasst das Verfahren zur Herstellung von Verbindungen der Formel (I) die Kultivierung von *Coniochaeta ellipsoidea* Udagawa, DSM 13856, seiner Mutanten und/oder Varianten unter aeroben Bedingungen in einem jeweils eine oder mehrere Kohlenstoffquellen, Stickstoffquellen, anorganische Salze und gegebenen-falls Spurenelemente enthaltenden Kulturmedium.

Anstelle des Stammes DSM 13856 können auch deren Mutanten und/oder Varianten eingesetzt werden, soweit sie die erfindungsgemäßen Verbindungen synthetisieren.

Mutanten sind zur selben Species gehörenden Organismen, die sich in ihren Genen unterscheiden und damit verschiedene Genotypen darstellen ("Genotype: The genetic constitution of an organism, usually in respect to one gene or a few genes relevant in a particular context", McGraw-Hill, Dictionary of scientific and technical terms, McGraw-Hill Book Company, N. Y. 1978, Seite 672).

Die Herstellung von Mutanten ist unter anderem beschrieben in Brock et al. "Biology of Microorganisms", Prentice Hall, S. 238-247 (1994), wonach beispielsweise physikalische Mittel, z.B. Bestrahlung mit Ultraviolett- oder Röntgenstrahlen, oder durch chemische Mutagene, wie z.B. Ethylmethansulfonat (EMS); 2-Hydroxy-4-methoxy-benzophenon (MOB) oder N-Methyl-N'-nitro-N-nitrosoguanidin (MNNG) Mutationen induzieren können.

Varianten im Sinne der Erfindung unterscheiden sich bei identischem Genom im Phenotyp vom Wildtyp ("Phenotype: The observable characters of an organism", McGraw-Hill, Dictionary of scientific and technical terms, McGraw-Hill Book Company, N. Y. 1978, Seite 1199). Mikroorganismen haben die Fähigkeit, in Abhänigigkeit von ihrer Umgebung verschiedenen Phenotypen auszubilden: "Microorganisms have the ability to adapt to enviromental changes. This adaptive capacity is the reason for the observed physiological flexibility. In phenotypic adaptation, all cells of a population are involved. This type of change is not genetically conditioned. It is a modification that under altered conditions is reversible" (H. Stolp, "Microbial ecology: organisms, habitats, activities", Cambridge University Press, Cambridge, GB, 1988, Seite 180).

Das Screening nach Mutanten und Varianten, die die erfindungsgemäßen Verbindungen produzieren, kann durch Bestimmung der biologischen Aktivität des in der Kulturbrühe angehäuften Wirkstoffes, oder durch Hochleistungsflüssigkeits-Chromatographie (HPLC-Bestimmung) erfolgen.

Als Kohlenstoffquellen für die Fermentation eignen sich beispielsweise assimilierbare Kohlenhydrate und Zuckeralkohole, wie Glukose, Laktose, Saccharose oder D-Mannit sowie kohlenhydrathaltige Naturprodukte, wie z.B. Malzextrakt oder Hefextrakt. Als stickstoffhaltige Nährstoffe kommen beispielsweise Aminosäuren, Peptide und Proteine sowie deren Abbauprodukte, wie Casein, Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate, insbesondere aber auch synthetisch bzw. biosynthetisch gewonnene Peptide in Betracht. Als anorganische Salze kann die Nährlösung beispielsweise Chloride, Carbonate, Sulfate oder Phosphate der Alkali- oder Erdalkalimetalle, enthalten, und als Spurenelemente beispielweise Eisen, Zink, Kobalt, Molybden, Bor, Vanadium und Mangan.

Bevorzugt enthält das Kulturmedium Malzextrakt, Hefeextrakt, Glukose, Stärke, Haferflocken und/oder Glycerin, besonders bevorzugt in Mengenanteilen von 0,05 bis 5 %, bevorzugt 1 bis 2 % Malzextrakt, 0,05 bis 3 %, bevorzugt 0,05 bis 1 % Hefeextrakt, 0,2 bis 5 %, bevorzugt 0,5 bis 2 % Glucose und 0,5 bis 3 %, bevorzugt 1.5% bis 3 % Haferflocken, jeweils bezogen auf das Gewicht der gesamten Nährlösung.

In dieser Nährlösung bildet *Coniochaeta ellipsoidea* Udagawa, DSM 13856, ein Gemisch aus den erfindungsgemäßen Verbindungen der Formel (I). Je nach Zusammensetzung der Nährlösung kann der mengenmäßige Anteil eines oder mehrerer der erfindungsgemäßen Coniosulfide variieren. Außerdem kann durch die Medienzusammensetzung die Synthese einzelner Coniosulfide gesteuert werden, so daß ein Coniosulfid gar nicht bzw. in einer Menge unterhalb der Nachweisgrenze vom Mikroorganismus hergestellt wird.

Die Kultivierung des Mikroorganismus erfolgt bevorzugt aerob, also beispielsweise submers unter Schütteln oder Rühren in Schüttelkolben oder Fermentern oder auf Festmedium, gegebenenfalls unter Einführen von Luft oder Sauerstoff. Die Kultivierung kann in einem Temperaturbereich von etwa 15 bis 35°C, vorzugsweise bei etwa 20 bis 35°C, insbesonders bei 25 bis 30°C durchgeführt werden. Der pH-Bereich der Kultivierung kann zwischen 3 und 10 liegen, vorzugsweise zwischen 6.5 und 7.5. Man kultiviert den Mikroorganismus unter diesen Bedingungen im allgemeinen über einen Zeitraum von 48 bis 960 Stunden, vorzugsweise 72 bis 720 Stunden. Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10-1:100, überimpft werden. Die Vorkultur erhält man z.B., indem man das Mycel in eine Nährlösung überimpft und etwa 20 bis 120 Stunden, bevorzugt 48 bis 72 Stunden, wachsen läßt. Das Mycel kann beispielsweise erhalten werden, indem man den Stamm etwa 1 bis 40 Tage, bevorzugt 21 bis 35 Tage, auf einem festen oder flüssigen Nährboden, beispielsweise Hefe-Malz-Agar, Haferflocken-Agar oder Kartoffeldextrose-Agar, wachsen läßt.

Der Fermentationsverlauf und die Bildung der erfindungsgemäßen Verbindungen kann entsprechend dem Fachmann bekannten Methoden, wie z.B. durch Testen der biologischen Aktivität in Bioassays oder durch chromatographische Methoden wie Dünnschichtchromatographie (DC) oder Hochleistungsflüssigkeits-Chromatographie (HPLC) verfolgt werden.

Der Pilz *Coniochaeta ellipsoidea* Udagawa, DSM 13856, kann die Coniosulfide durch eine Oberflächen- oder Standkultur auf festen Nährböden bilden. Feste Nährböden werden durch Zugabe zum Beispiel von Agar oder Gelatine zu wäßrigen Nährmedien hergestellt. Es ist aber auch möglich, die Coniosulfide durch Fermentation des Pilzes Coniochaeta ellipsoidea Udagawa im Submersverfahren, d.h. in wäßriger Aufschlämmung zu gewinnen. Die Coniosulfide können sowohl im Mycel als auch im Kulturfiltrat vorkommen, gewöhnlich befindet sich die Hauptmenge in der Zellmasse. Es ist deshalb zweckmäßig, die Fermentationslösung durch Filtration oder Zentrifugation zu trennen. Das Filtrat wird mit einem Adsorptionsharz als feste Phase extrahiert. Das Mycel, aber auch die Oberflächenkultur wird mit einem Lösungsmittel extrahiert, daß mit Wasser mischbar ist, beispielsweise mit Acetonitril, n-Propanol oder iso-Propanol, vorzugsweise mit Methanol oder Aceton, oder mit einem mit Wasser nicht mischbaren Lösungsmittel, beispielsweise mit Chloroform, Dichloromethan oder vorzugsweise mit tert-Butanol oder Ethylacetat. Vorzugsweise werden Verbindungen der Formel (I) mit Methanol oder Propanol-2 extrahiert. Die Extraktionen können in einem weiten pH-Bereich durchgeführt werden, es ist jedoch zweckmäßig, im neutralen oder schwach sauren Milieu, vorzugsweise zwischen pH 3 und 8 zu arbeiten. Die Extrakte können im Vakuum konzentriert und getrocknet werden.

Eine Methode der Isolierung der erfindungsgemäßen Coniosulfide ist die Lösungsverteilung zwischen Lösungsmitteln und/oder Absorptionsverteilung an Feststoffen unterschiedlicher Polarität in an sich bekannter Weise.

Eine andere Methode der Reinigung ist die Chromatographie an Adsorptionsharzen wie z.B. an Diaion^{®} HP-20 (Mitsubishi Casei Corp., Tokyo), an Amberlite^{®} XAD 7 (Rohm and Haas, USA), an Amberchrom^{®} CG, (Toso Haas, Philadelphia, USA) oder an ähnlichen. Geeignet sind darüber hinaus zahlreiche Reverse-Phase Träger, z.B. RP₈ und RP₁₈, wie sie z.B. im Rahmen der Hochdruckflüssigkeits-Chromatographie (HPLC) allgemein bekannt geworden sind.

Eine weitere Reinigungsmöglichkeit für die erfindungsgemäßen Coniosulfide besteht in der Verwendung von sogenannten Normal-Phasen-Chromatographie-Trägern, wie beispielweise Kieselgel oder Al₂O₃, in an sich bekannter Weise.

Ein alternatives Isolierungsverfahren ist die Verwendung von Molekularsieben, wie z.B. Fractogel^{®} TSK HW-40, Sephadex^{®} G-25 und andere, in an sich bekannter Weise. Es ist darüber hinaus auch möglich, aus angereichertem Material die Coniosulfide durch Kristallisation zu gewinnen. Geeignet hierzu sind z.B. organische Lösungsmittel und ihre Gemische, wasserfrei oder mit Wasserzusatz. Ein zusätz-liches Verfahren zur Isolierung und Reinigung der erfindungsgemäßen Verbindungen besteht in der Verwendung von Anionenaustauschern, vorzugsweise im pH-Bereich von 4 bis 10 und Kationenaustauschern vorzugsweise im pH-Bereich von 2 bis 5. Besonders geeignet ist hierfür die Verwendung von Pufferlösungen, denen man Anteile von organischen Lösungsmitteln hinzugefügt hat.

Es wurde gefunden, daß die erfindungsgemäßen Verbindungen der Formel (I), (II), (III), (IV) und (V) starke Hemmung der Amyloid Precursor Protein - FE65-Wechselwirkung aufweisen, sie eignen sich daher zur Behandlung und Prophylaxe von degenerativen Neuropathien und der Alzheimer'schen Krankheit. Tabelle 1 faßt ausgewählte Hemmkonstanten beispielhaft zusammen.

**Tabelle 1: Die Hemmung der Amyloid Precursor Protein - FE65 - Wechselwirkung durch Coniosulfid A-D:**

| | |
|---|---|
| Coniosulfid A (Formel (II) | IC₅₀ = 2,3 µM |
| Coniosulfid B (Formel (III) | IC₅₀ = 2,6 µM |
| Coniosulfid C (Formel (IV) | IC₅₀ = 3,8 µM |
| Coniosulfid D (Formel (V) | IC₅₀ = 3,8 µM |

Ein weiterer Gegenstand der vorliegenden Erfindung ist somit ein Arzneimittel mit einem Gehalt an mindestens einer Verbindung der Formel (I), vorzugsweise einer Verbindung der Formel (II) bis (V), und einem oder mehreren physiologisch geeigneten Träger und/oder Hilfsstoff.

Ferner ist ein Gegenstand der vorliegenden Erfindung die Verwendung einer Verbindung der Formel (I), vorzugsweise einer Verbindung der Formel (II) bis (V), als Arzneimittel.

Ein weiterer Gegenstand der Erfindung ist die Verwendung einer Verbindung der Formel (I), vorzugsweise einer Verbindung der Formel (II) bis (V), zur Herstellung von Arzneimitteln zur Behandlung und/oder zur Prophylaxe von degenerativen Neuropathien, vorzugsweise von Altersdemenz oder der Alzheimer'schen Krankheit.

Das besagte Arzneimittel wird durch Mischung von mindestens einer Verbindung der Formel (I) mit mindestens einem physiologischen geeigneten Träger und/oder Hilfsstoff hergestellt und in eine geeignete Darreichungsform gebracht.

Die erfindungsgemäßen Arzneimittel können enteral (oral), parenteral (intramuskulär oder intravenös), rektal oder lokal (topisch) angewendet werden. Sie können in Form von Lösungen, Pulvern, Tabletten, Kapseln einschließlich Mikrokapseln, Salben, Cremes, Gel, oder Suppositorien verabreicht werden. Als physiologisch geeignete Träger oder Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 1000, vorzugsweise 0.2 - 100 mg/kg Körpergewicht verabreicht. Sie werden zweckmäßig in Dosierungseinheiten verabreicht, die beispielsweise die wirksame Tagesmenge der erfindungsgemäßen Verbindungen, z.B. 30 - 3000, vorzugsweise 50 - 1000 mg enthalten.

Die folgenden Beispiele sollen der näheren Erläuterung der Erfindung dienen, ohne die Breite der Erfindung in irgendeiner Weise begrenzen zu wollen.

### Beispiel 1 Herstellung einer Glycerinkultur von Coniochaeta ellipsoidea, DSM 13856

30 mL Nährlösung (Malzextrakt 2,0%, Hefeextrakt 0,2%, Glucose 1,0 % (NH₄)₂HPO₄ 0,05 %, pH 6,0) in einem sterilen 100 mL Erlenmeyerkolben werden mit dem Stamm *Coniochaeta ellipsoidea* Udagawa, DSM 13856, beimpft und 6 Tage bei 25 °C und 140 UpM auf einer rotierenden Schüttelmaschine inkubiert. 1,5 ml dieser Kultur werden anschließend mit 2,5 ml 80 %igem Glycerin verdünnt und bei -135°C gelagert.

### Beispiel 2 Herstellung einer Vorkultur im Erlenmeyerkolben von Coniochaeta ellipsoidea Udagawa, DSM 13856

100 mL Nährlösung (Malzextrakt 2,0 %, Hefeextract 0,2 %, Glucose 1,0 % (NH₄)₂HPO₄ 0,05 %, pH 6,0) in einem sterilen 300 mL Erlenmeyerkolben werden mit dem Stamm *Coniochaeta ellipsoidea* Udagawa, DSM 13856, beimpft und 4 Tage bei 25 °C und 140 UpM auf einer rotierenden Schüttelmaschine inkubiert. 2 ml dieser Vorkultur werden anschließend für die Herstellung der Hauptkulturen verwendet.

### Beispiel 3 Herstellung einer Hauptkultur von Coniochaeta ellipsoidea Udagawa, DSM 13856, auf Festmedium Platten.

100 sterile 22 x 22 cm² Platten werden jeweils mit 200 mL der folgenden Nährlösung 20 g/L Malzextrakt, 20 g/L Haferflocken, 2% Agar und pH 7.0 gegossen. Diese Platten werden mit 2 mL einer Vorkultur beimpft und bei 25 °C inkubiert. Die maximale Produktion einer oder mehrerer Verbindungen der erfindungsgemäßen Coniosulfiden ist nach ca. 676 Stunden erreicht.

### Beispiel 4: Isolierung der Coniosulfide.

Es wurden 100 Stück der nach Beispiel 3 gewonnenen Agarkulturen von *Coniochaeta ellipsoidea* Udagawa, DSM 13856, gefriergetrocknet. Sie ergaben 499 g Kultur-Lyophilisat. Das Lyophilisat wurde dreimal mit je 20 Liter Methanol extrahiert, im Vakuum bei Raumtemperatur konzentriert und anschließend im Hochvakuum gefriergetrocknet. Daraus resultierten 79,8 g Rohextrakt. Das lyophilisierte Rohextrakt wurde in 25% Methanol in 0,050mol/L Ammoniumacetat gelöst und über eine Säule (50 x 300mm), gefüllt mit 0,5 Liter MCI Gel CHP20P (Mitsubishi Chemical Industries, Tokyo, Japan), gepumpt. Die Säule wurde zunähst mit 1 L 25 Vol-% Acetonitril in 75 Vol- % 50 mM wässrigem Ammoniumacetat- Puffer gewaschen und anschließend mit einem linearen Gradienten von 44 Vol-% bis 100 Vol-% Acetonitril in 50 mM Ammoniumacetat eluiert. Nach HPLC-DAD-Analyse wurde die Coniosulfid-haltige Fraktion am Rotationsverdampfer im Vakuum bei 38 °C Badtemperarur konzentriert und gefriergetrocknet. Die Ausbeute an lyophilisiertem Coniosulfid-Gemisch betrug 0,26 g. Dieses Coniosulfid-Gemisch wurde in Methanol gelöst und auf eine präparative HPLC Säule, gefüllt mit LiChrospher RP-18e (10 µm, E. Merck, Darmstadt, Deutschland) (Säulenmaße: 25 x 250 mm² + Vorsäule 25 x 10 mm²) aufgetragen. Die RP-Säule wurde zunächst mit 30 Vol-% Acetonitril in 50 mM Ammoniumacetat- Puffer eluiert mit einer Flußrate von 50 mL pro Minute und dann, ab Fr.14, mit einem linearen Gradienten von 30 Vol-% bis 100 Vol-% Acetonitril in 50 mM Ammoniumacetat-Puffer. Die Fraktionen (je 25 ml) wurden analytisch im HPLC-DAD-System untersucht. Die Fraktion 30 enthielt das Coniosulfid A, Fraktion 33 das Coniosulfid B, Fraktion 28 das Coniosulfid C und Fraktion 31 das Coniosulfid D.

### Beispiel 5: Endreinigung des Coniosulfids A.

Das angereicherte Coniosulfid A (Fraktion 30), gewonnen nach Beispiel 4, wurde auf einer LiChrospher^{®} 100 RP-18e-HPLC-Säule (5 µm, Breite x Höhe = 1 cm x 25 cm) mit 50 % Acetonitril in 10 mM Essigsäure aufgetrennt. Fluß: 10 mL/Min. Die durch analytische HPLC (siehe Beispiel 10) untersuchten Fraktionen wurden entsprechend ihrem Coniosulfid A-Gehalt zusammen gefaßt, im Vakuum eingeengt und gefriergetrocknet. Sie ergeben 8 mg Coniosulfid A in 98 %iger Reinheit.

### Beispiel 6: Charakterisierung des Coniosulfids A.

Die physikalisch-chemischen sowie spektroskopischen Eigenschaften des Coniosulfids A lassen sich wie folgt zusammenfassen:
Aussehen:
   Farblose bis hellgelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.
   Summenformel: C₂₂H₃₄O₄N₂S;
   Molekulargewicht: 422,59 Da;
   ¹H- und ¹³C-NMR: siehe Tabelle 2;
   UV-Maximum: 288 nm
Massenspektrometrische Untersuchungen:
   Coniosulfid A wurde die Masse 422 zugeordnet aufgrund folgender Befunde: ESI⁺-Spektrum lieferte Peaks bei 423 amu (M+H)⁺, 445 (M+Na)⁺ und 461 (M+K)⁺.
   Mit einem FTICR-Massenspektrometer (Fourier Transform Ion Cyclotron Resonance) wurde u.a. ein Peak bei 423.2314 amu beobachtet. Der Messwert entspricht der für (M+H)⁺ = C₂₂H₃₅O₄N₂S = 423.2312 berechneten Masse.
   MS/MS-Experimente mit einem FTICR-Massenspektrometer führten zu folgenden Fragmentierungen: ESI⁺-Modus: 423 amu (M+H)⁺ zu 324 amu (-C₄H₅NO₂), 280 amu (-C₅H₅NO₄), 237 amu (-C₇H₁₀N₂O₄), 219 amu (-C₁₅H₂₄), 205 amu (-C₇H₁₀N₂O₄S), 100 amu (C₄H₆O₂N+), und kleinere Fragmente.

**Tabelle 2: Chemische Verschiebungen von Coniosulfid A in DMSO bei 300 K.**

| Position | ¹H | ¹³C |
|---|---|---|
| 1 | 1,63 | 25,44 |
| 2 | - | 130,58 |
| 2-Me | 1,55 | 17,51 |
| 3 | 5,06 | 124,08 |
| 4 | 2,02 | 26,14 |
| 5 | 1,93 | 38,98 |
| 6 | - | 134,66 |
| 6-Me | 1,56 | 15,75 |
| 7 | 5,08 | 123,52 |
| 8 | 2,06 | 25,96 |
| 9 | 2,01 | 39,0 (breit) |
| 10 | - | 139,78 |
| 10-Me | 1,68 | 15,88 |
| 11 | 5,25 | 119,57 |
| 12 | 3,50 | 30,55 |
| 13 | 7,38 | 137,6 (breit) |
| 14 | - | 122,4 (breit) |
| 15 | - | 163,69 |
| 16 | 8,99 | - |
| 17 | - | 167,28 |
| 18 | 3,78 | 41,51 |
| 19 | 8,07 | - |
| 20 | - | 169,36 |
| 21 | 1,85 | 22,39 |

### Beispiel 7: Endreinigung und Charakterisierung des Coniosulfids B.

Das angereicherte Coniosulfid B (Fraktion 33), gewonnen nach Beispiel 4, wurde auf einer LiChrospher^{®} 100 RP-18e-HPLC-Säule (5 µm, Breite x Höhe = 1 cm x 25 cm) mit 50 % Acetonitril in 10 mM Essigsäure aufgetrennt. Fluß: 10 mL/Min. Die durch analytische HPLC (siehe Beispiel 10) untersuchten Fraktionen wurden entsprechend ihrem Coniosulfid B-Gehalt zusammen gefaßt, im Vakuum eingeengt und gefriergetrocknet. Sie ergeben 6 mg Coniosulfid B in 97 %iger Reinheit.

Die physikalisch-chemischen sowie spektroskopischen Eigenschaften des Coniosulfids B lassen sich wie folgt zusammenfassen:
Aussehen:
   Farblose bis hellgelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.
   Summenformel: C₂₃H₃₆O₄N₂S;
   Molekulargewicht: 436,62 Da;
   ¹H- und ¹³C-NMR: siehe Tabelle 3;
   UV-Maximum: 288 nm
Massenspektrometrische Untersuchungen:
   Coniosulfid B wurde die Masse 436 zugeordnet aufgrund folgender Befunde: ESI⁺-Spektrum lieferte Peaks bei 437 amu (M+H)⁺, 459 amu (M+Na)⁺ und 475 amu (M+K)⁺.
   Hochauflösung des Quasi-Molekülions: Mit einem FTICR-Massenspektrometer wird im ESI⁺-Modus u.a. ein Peak bei 437.2472 amu beobachtet. Der Messwert stimmt mir der für (M+H)⁺ = C₂₃H₃₇O₄N₂S = 437.2469 amu berechneten überein.
   MS/MS-Experimente mit einem FTICR-Massenspektrometer führen zu folgenden Fragmentierungen:
   ESI⁺-Modus: 437 amu zu 338 amu (-C₄H₅NO₂), 321 amu (-C₄H₈N₂O₂), 294 (-C₅H₅NO₄), 251.amu (-C₇H₁₀N₂O₄), 219.04 amu (-C₁₆H₂₆), 219.21 (-C₇H₁₀N₂O₄S), 100 amu (C₄H₆O₂N+) und kleinere Fragmente.

**Tabelle 3: Chemische Verschiebungen von Coniosulfid B in DMSO bei 300 K.**

| Position | ¹H | ¹³C |
|---|---|---|
| 1 | 1,59 | 19,83, ^{a)} |
| 2 | - | 123,29 |
| 2-Me | 1,58 | 20,37, ^{a)} |
| 3 | - | 135,07 |
| 3-Me | 1,58 | 18,20 |
| 4 | 2,04 | 33,05 |
| 5 | 1,94 | 37,69 |
| 6 | - | 127,12 |
| 6-Me | 1,58 | 15,84 |
| 7 | 5,08 | 123,35 |
| 8 | 2,04 | 26,01 |
| 9 | 2,01 | 38,96 |
| 10 | - | 139,82 |
| 1 0-Me | 1,68 | 15,88 |
| 11 | 5,25 | 119,53 |
| 12 | 3,50 | 30,55 |
| 13 | 7,37 | 137,6 (breit) |
| 14 | - | 122,4 (breit) |
| 15 | - | 163,70 |
| 16 | 8,99 | - |
| 17 | - | 167,29 |
| 18 | 3,78 | 41,52 |
| 19 | 8,07 | - |
| 20 | - | 169,36 |
| 21 | 1.85 | 22,39 |

| | | |
|---|---|---|
| ^{a)} Die beiden Methylgruppen konnten nicht eindeutig zugeordnet werden. | | |

### Beispiel 8. Endreinigung und Charakterisierung des Coniosulfids C.

Das angereicherte Coniosulfid C (Fraktion 28), gewonnen nach Beispiel 4, wurde auf einer LiChrospher^{®} 100 RP-18e-HPLC-Säule (5 µm, Breite x Höhe = 1 cm x 25 cm) mit 50 % Acetonitril in 10 mM Essigsäure aufgetrennt. Fluß: 10 mL/Min. Die durch analytische HPLC (siehe Beispiel 10) untersuchten Fraktionen wurden entsprechend ihrem Coniosulfid C-Gehalt zusammen gefaßt, im Vakuum eingeengt und gefriergetrocknet. Sie ergeben 18 mg Coniosulfid C in 98 %iger Reinheit.

Die physikalisch-chemischen sowie spektroskopischen Eigenschaften des Coniosulfids C lassen sich wie folgt zusammenfassen:
Aussehen:
   Farblose bis hellgelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.
   Summenformel: C₂₄H₃₇O₅N₃S,
   Molekulargewicht: 479,64 Da;
   ¹H- und ¹³C-NMR: siehe Tabelle 4;
   UV-Maximum: 288 nm
Massenspektrometrische Untersuchungen:
   Coniosulfid C wurde die Masse 479 amu zugeordnet aufgrund folgender Befunde: ESI⁺-Spektrum lieferte Peaks bei 502 amu (M+Na)⁺ und 518 amu (M+K)⁺.
   ESI⁻Spektrum lieferte u.a. einen Peak bei 478 amu (M-H)⁻.
   Mit einem FTICR-Massenspektrometer wurde im ESI⁻-Modus u.a. ein Peak bei 478.23810 amu beobachtet. Der Messwert stimmt mit der für (M-H)⁻ = C₂₄H₃₆O₅N₃S = 478.23812 amu berechneten überein.
   MS/MS-Experimente mit einem FTICR-Massenspektrometer führte zu folgenden Fragmentierungen: ESI⁻-Modus:
   478 amu (M-H)⁻ zu 434 amu (-CO₂) 416 amu (-H₂CO₃) 230 amu (-C₁₅H₂₄CO₂), 229 amu (-C₁₅H₂₅CO₂), 196 amu (C₈H₁₀N₃O₃⁻), 155 amu (C₆H₇N₂O₃⁻), 154 amu (C₆H₆N₂O₃⁻ ) und kleinere Fragmente.

**Tabelle 4: Chemische Verschiebungen von Coniosulfid C in DMSO bei 300 K.**

| Position | ¹H | ¹³C |
|---|---|---|
| 1 | 1,63 | 25,44 |
| 2 | - | 130,58 |
| 2-Me | 1,55 | 17,51 |
| 3 | 5,06 | 124,08 |
| 4 | 2,02 | 26,14 |
| 5 | 1,93 | 39,2 (breit) |
| 6 | - | 134,67 |
| 6-Me | 1,56 | 15,75 |
| 7 | 5,09 | 123,53 |
| 8 | 2,05 | 25,97 |
| 9 | 2,01 | 38,99 |
| 10 | - | 139,84 |
| 10-Me | 1,68 | 15,89 |
| 11 | 5,26 | 119,55 |
| 12 | 3,50 | 30,54 |
| 13 | 7,39 | 137,9 (breit) |
| 14 | - | 122,3 (breit) |
| 15 | - | 163,65 |
| 16 | 8,98 | - |
| 17 | - | 167,04 |
| 18 | 3,81 | 41,47 |
| 19 | 8,07 | - |
| 20 | - | 169,20 |
| 21 | 3,70 | 42,00 |
| 22 | 8,10 | - |
| 23 | - | 169,57 |
| 24 | 1,85 | 22,43 |

### Beispiel 9: Endreinigung und Charakterisierung des Coniosulfids D.

Das angereicherte Coniosulfid D (Fraktion 31), gewonnen nach Beispiel 4, wurde auf einer LiChrospher^{®} 100 RP-18e-HPLC-Säule (5 µm, Breite x Höhe = 1 cm x 25 cm) mit 35 % Acetonitril in 10 mM Ammoniumacetat-Puffer aufgetrennt. Fluß: 5 mL/Min. Die durch analytische HPLC (siehe Beispiel 10) untersuchten Fraktionen wurden entsprechend ihrem Coniosulfid D-Gehalt zusammen gefaßt, im Vakuum eingeengt und gefriergetrocknet. Sie ergeben 18 mg Coniosulfid D in 97 %iger Reinheit.

Die physikalisch-chemischen sowie spektroskopischen Eigenschaften des Coniosulfids D lassen sich wie folgt zusammenfassen:
Aussehen:
   Farblose bis hellgelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.
   Summenformel: C₂₅H₃₉O₅N₃S;
   Molekulargewicht: 493,67 Da;
   ¹H- und ¹³C-NMR: siehe Tabelle 5;
   UV-Maximum: 288 nm
Massenspektrometrische Untersuchungen:
   Coniosulfid D wurde die Masse 493 zugeordnet aufgrund folgender Befunde: ESI⁺-Spektrum lieferte Peaks bei 494 amu (M+H)⁺ und 516 amu (M+Na)⁺. ESI⁻ Spektrum liefert u.a. einen Peak bei 492 amu (M-H)⁻.

Mit einem FTICR-Massenspektrometer wurde u.a. ein Peak bei 494.2687 amu beobachtet. Der Messwert stimmte mit dem für (M+H)⁺ = C₂₅H₄₀O₅N₃S = 494.2683 amu berechneten überein (0.7 ppm Abweichung).

MS/MS-Experimente mit einem FTICR-Massenspektrometer führen zu folgenden Fragmentierungen:
ESI⁺-Modus: 494 (M+H)⁺ zu 476 (-H₂O), 395 (-C₄H₅NO₂), 338 (-C₆H₈N₂O₃), 294 (-C₇H₈N₂O₆), 157 (-C₁₉H₃₁NO₂S).

ESI⁻-Modus: 492 zu 448 (-CO₂), 430 (-CH₂O₃), 230 (-C₁₆H₂₆CO₂), 229 (-C₁₆H₂₇CO₂), 196 (C₈H₁₀N₃O₃⁻), 155 (C₆H₇N₂O₃⁻), 154 (C₆H₆N₂O₃⁻).

**Tabelle 5: Chemische Verschiebungen von Coniosulfid D in DMSO bei 300 K.**

| Position | ¹H | ¹³C |
|---|---|---|
| 1 | 1,60 | 19,83, ^{a)} |
| 2 | - | 123,28 |
| 2-Me | 1,58 | 20,36, ^{a)} |
| 3 | - | 134,98 |
| 3-Me | 1,58 | 18,20 |
| 4 | 2,04 | 33,04 |
| 5 | 1,94 | 37,70 |
| 6 | - | 127,12 |
| 6-Me | 1,58 | 15,84 |
| 7 | 5,09 | 123,47 |
| 8 | 2,04 | 26,06 |
| 9 | 1,99 | 39,03 |
| 10 | - | 138,73 |
| 10-Me | 1,65 | 15,88 |
| 11 | 5,23 | 120,07 |
| 12 | 3,32 | 31,04 |
| 13 | 6,86 | 125,6 (breit) |
| 14 | - | -, ^{b)} |
| 15 | - | 164,8 (breit) |
| 16 | 8,8 (breit) | - |
| 17 | - | 165,5 (breit) |
| 18 | 3,77 | 42,0 (breit) |
| 19 | 8,18 | - |
| 20 | - | 169,22 |
| 21 | 3,71 | 42,00 |
| 22 | 8,17 | - |
| 23 | - | 169,53 |
| 24 | 1,85 | 22,42 |

| | | |
|---|---|---|
| ^{a)} Die beiden Methylgruppen konnten nicht eindeutig zugeordnet werden. ^{b)} Für C14 wurde kein Signal beobachtet (extreme Linienverbreiterung). | | |

### Beispiel 10: Hochdruckflüssigkeitschromatographie (HPLC) der Coniosulfide A-D.

| | |
|---|---|
| Säule: | Superspher 100 RP-18e^{®}, 250-4, mit Vorsäule, |
| Mobile Phase: | 55 % Acetonitril in 0.1 % Phosphorsäure, |
| Flußgeschwindigkeit: | 1 mL pro-Minute, |

Detektion durch UV-Absorption bei 210 nm.
Es wurden folgende Retentionszeit gefunden:

| | |
|---|---|
| Coniosulfid A | 14,8 Minuten; |
| Coniosulfid B | 20,1 Minuten; |
| Coniosulfid C | 10,6 Minuten; |
| Coniosulfid D | 14,2 Minuten; |

### Beispiel 11: Hemm-Test der APP - FE65 - Wechselwirkung.

Reagenzien und Bezugsquellen:

| | |
|---|---|
| HEPES, | Sigma, H-9136, |
| NaCl, | Riedel de Haen, 31434, |
| EDTA, | Sigma, E-1644, |
| CHAPS, | Sigma, C-5849, |
| KF, | Sigma, P-1179, |
| BSA, | Sigma, A-9647 |
| Monoclonale Anti GST Antikörper | |
| markiert mit XL665, | CIS bio Intern., D11/459/12/47270; |
| Streptavidin markiert mit Eu-cryptat, | CIS bio Intern., D11/450/12/47269; |
| 32mer cAPP, | Sigma, E2533; |
| Biotin-cAPP, | Aventis SA, Vitry. |

### Puffer A:

10 mM HEPES, pH 7.2 + 150 mM NaCl + 3.4 mM EDTA + 0.184 g/L CHAPS.

### Puffer B:

100 mM HEPES, pH 7.0 + 400 mM KF + 133 mM EDTA + 1 g/L BSA.

### Durchführung:

1 µl der zu prüfenden Lösung wurde zusammen mit 30 µM 32mer cAPP in Puffer A in eine Mikrotiterplatte pipettiert. Dann wurde 1 µl Biotin-cAPP (10nM, gelöst in Puffer A) hinzugefügt. Nach 15 minütigem Inkubieren setzte man 1 µL GST-PTB2 (20 nM in Puffer A) hinzu und inkubierte erneut 15 Minuten lang. Dann wurden 4 µl Antikörpergemisch (Streptavidin markiert mit Eu-cryptat, 2 ng pro well; Monoklonaler Anti GST Antikörper markiert mit XL665, 25 ng in Puffer B) hinzugefügt.
Nach 30 Minuten bei Raumtemperatur wurde in einem Tecan Ultra Photometer das Emissions-Signal der Energie-Übertragung sowie das Europium-Signal gemessen bei 665nm und 615 nm, nachdem die Testlösung mit Licht der Wellenlänge 340 nm angeregt worden ist.

Für die Hemmung der Amyloid Precursor Protein - FE65 - Wechselwirkung wurden die in Tabelle 1 genannten IC₅₀-Werte gefunden.

## Patentansprüche

1. Verbindung der Formel (I) 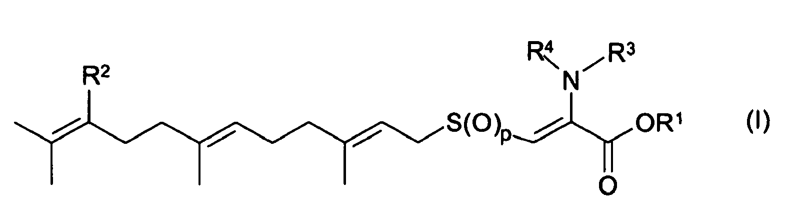 wobei
R¹
1.0 H, oder
2.0 ein Rest ausgewählt aus -C₁-C₆-Alkyl, -C₂-C₆-Alkenyl, -C₂-C₆-Alkinyl oder -C₆-C₁₀-Aryl, der ein- oder zweifach substituiert sein kann durch
2.1 -OH,
2.2 =O,
2.3 -O-(C₁-C₆-Alkyl),
2.4 -O-(C₂-C₆-Alkenyl),
2.5 -C₆-C₁₀-Aryl,
2.6 -NH-C₁-C₆-Alkyl,
2.7 -NH-C₂-C₆-Alkenyl,
2.8 -NH₂ oder
2.9 Fluor, Chlor, Brom oder lod,
worin die Substituenten 2.3 bis 2.8 auch noch weiter substituiert sein können durch -CN, -Amid oder -Oxim-Funktionen, oder
3.0 -(C₁-C₄-Alkyl)-(C₆-C₁₀-Aryl), oder
4.0 -NH₂, -NH-(C₁-C₆-Alkyl), -NH-(C₂-C₆-Alkenyl) ist,
R² H, Methyl, Ethyl, n-Propyl, iso-Propyl, n-Butyl oder iso-Butyl,
R³ ein aus 1 bis 10 Aminosäuren bestehender Rest, der N-terminal geschützt sein kann,
R⁴ H, -C₁-C₆-Alkyl, -CO-(C₁-C₆-Alkyl), und
p eine ganze Zahl 0, 1 oder 2 bedeutet,
ausgenommen Verbindungen der Formel (I), in denen gleichzeitig
R¹ H,
R² H,
R³ -CO-CH₃,
R⁴ H, und
p 0 oder 1 bedeuten,
und/oder eine stereoisomere Form der Verbindung der Formel (I) und/oder Gemische diese Form in jedem Verhältnis,
und/oder ein physiologisch verträgliches Salz der Verbindung der Formel (I).

2. Verbindung der Formel (I) gemäß Anspruch 1, wobei
R⁴ H bedeutet, und
R¹, R², R³ und p wie in Anspruch 1 definiert sind.

3. Verbindung der Formel (I) gemäß Anspruch 1, wobei
R⁴ H, und
p 0 bedeutet, und
R¹, R² und R³ wie in Anspruch 1 definiert sind.

4. Verbindung der Formel (I) gemäß Anspruch 1, wobei
R² H oder Methyl,
R⁴ H,
p 0 bedeutet, und
R¹ und R³ wie in Anspruch 1 definiert sind.

5. Verbindung der Formel (I) gemäß Anspruch 1, wobei
R² H oder Methyl ist,
R³ ein aus 1 bis 2 Aminosäuren bestehender Rest ist, der N-terminal geschützt sein kann,
R⁴ H,
p 0 bedeutet, und
R¹ wie in Anspruch 1 definiert sind.

6. Verbindung der Formel (I) gemäß Anspruch 1, wobei
R¹ H,
R² H oder Methyl ist,
R³ ein aus 1 bis 2 Aminosäuren bestehender Rest ist, der N-terminal geschützt sein kann,
R⁴ H, und
p 0 bedeutet.

7. Verbindung der Formel (II) 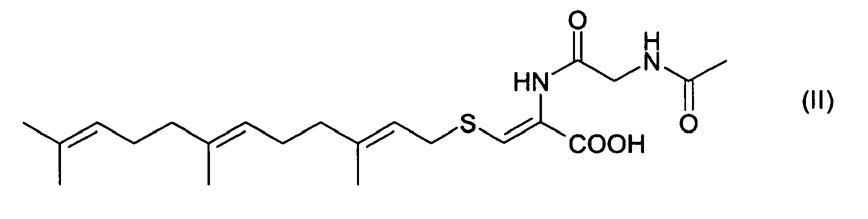 sowie deren physiologisch verträglichen Salze.

8. Coniosulfid A, eine Verbindung mit der Molekülformel C₂₂H₃₄O₄N₂S und charakterisiert durch die ¹H- und ¹³C-NMR-Daten entsprechend Tabelle 2, sowie physiologisch verträgliche Salze von Coniosulfid A.

9. Verbindung der Formel (III) : 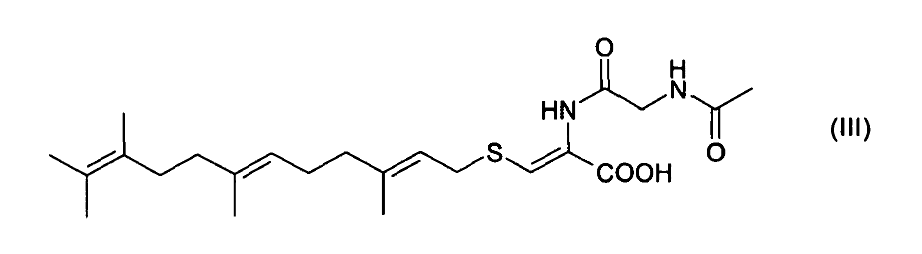 sowie deren physiologisch verträglichen Salze.

10. Coniosulfid B, eine Verbindung mit der Molekülformel C₂₃H₃₆O₄N₂S und charakterisiert durch die ¹H- und ¹³C-NMR-Daten entsprechend Tabelle 3, sowie physiologisch verträgliche Salze von Coniosulfid B.

11. Verbindung der Formel (IV) 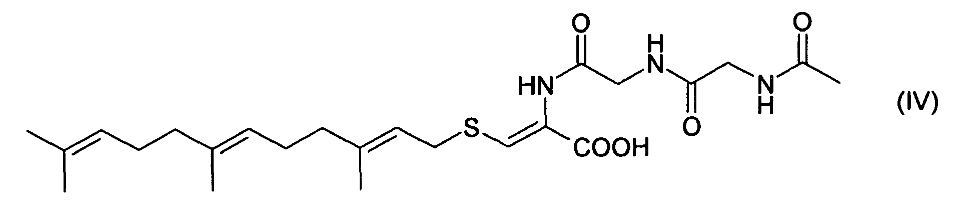 sowie deren physiologisch verträglichen Salze.

12. Coniosulfid C, eine Verbindung mit der Molekülformel C₂₄H₃₇O₅N₃S und charakterisiert durch die ¹H- und ¹³C-NMR-Daten entsprechend Tabelle 4, sowie physiologisch verträgliche Salze von Coniosulfid C.

13. Verbindung der Formel (V) 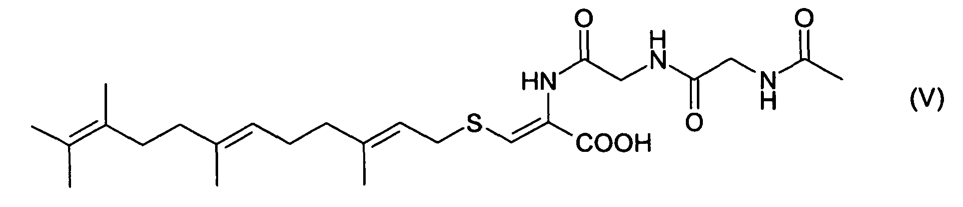 sowie deren physiologisch verträglichen Salze.

14. Coniosulfid D, eine Verbindung mit der Molekülformel C₂₅H₃₉O₅N₃S und charakterisiert durch die ¹H- und ¹³C-NMR-Daten entsprechend Tabelle 5, sowie physiologisch verträgliche Salze von Coniosulfid D.

15. Verbindung der Formel (I) nach einem der Ansprüche 1 bis 6, erhältlich durch Fermentierung von *Coniochaeta ellipsoidea* Udagawa, DSM 13856, oder einer ihrer Mutanten in einem Kulturmedium, bis sich eine Verbindung der Formel (I) in dem Kulturmedium anhäuft, und anschließender Isolierung der Verbindung der Formel (I), und optional Derivatisierung und/oder gegebenenfalls Überführung in ein pharmakologisch verträgliches Salz.

16. Verfahren zur Herstellung einer Verbindung der Formel (I), **dadurch gekennzeichnet, daß** der Mikroorganismus *Coniochaeta ellipsoidea* Udagawa, DSM 13856, oder eine Varianten oder Mutante von *Coniochaeta ellipsoidea* Udagawa, DSM 13856, in einem Kulturmedium fermentiert wird, bis sich eine Verbindung der Formel (I) in der Kulturbrühe anhäuft, die Verbindung der Formel (I) isoliert wird, und optional anschließend derivatisiert und gegebenfalls in ein physiologisch verträgliches Salz überführt wird.

17. Arzneimittel mit einem Gehalt an mindestens einer Verbindung gemäß einem oder mehreren der Ansprüche 1 bis 15 und mindestens einem physiologisch geeigneten Träger und/oder Hilfsstoff.

18. Verbindung gemäß einem oder mehreren der Ansprüche 1 bis 15 zur Verwendung als Arzneimittel.

19. Verbindung gemäß einem oder mehreren der Ansprüche 1 bis 15 zur Behandlung und Prophylaxe der Alzheimer'schen Krankheit.

20. Verfahren zur Herstellung eines Arzneimittels gemäß Anspruch 17, **dadurch gekennzeichnet, daß** man mindestens eine Verbindung gemäß einem oder mehreren der Ansprüche 1 bis 15 mit mindestens einem physiologischen geeigneten Träger und/oder Hilfsstoff in eine geeignete Darreichungsform bringt.

## Claims

1. A compound of the formula (I) 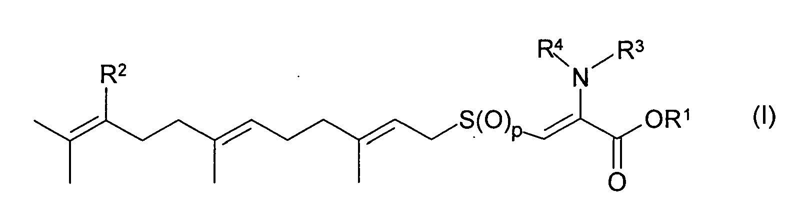 where
R¹ is
1.0 H, or
2.0 a radical selected from -C₁-C₆-alkyl, -C₂-C₆-alkenyl, -C₂-C₆-alkynyl or -C₆-C₁₀-aryl, which can be substituted once or twice by
2.1 -OH,
2.2 =O,
2.3 -O-(C₁-C₆-alkyl),
2.4 -O-(C₂-C₆-alkenyl),
2.5 -C₆-C₁₀-aryl,
2.6 -NH-C₁-C₆-alkyl,
2.7 -NH-C₂-C₆-alkenyl,
2.8 -NH₂ or
2.9 fluorine, chlorine, bromine or iodine,
in which the substituents 2.3 to 2.8 can also be still further
substituted by -CN, -amide or -oxime functions, or
3.0 -(C₁-C₄-alkyl)-(C₆-C₁₀-aryl), or
4.0 -NH₂, -NH-(C₁-C₆-alkyl), or -NH-(C₂-C₆-alkenyl),
R² is H, methyl, ethyl, n-propyl, isopropyl, n-butyl or iso-butyl,
R³ is a radical which is composed of from 1 to 10 amino acids and which can be N-terminally protected,
R⁴ is H, -C₁-C₆-alkyl, or -CO-(C₁-C₆-alkyl), and
p is an integer 0, 1 or 2,
with the exception of compounds of the formula (I) in which, at the same time,
R¹ is H,
R² is H,
R³ is -CO-CH₃,
R⁴ is H, and
p is 0 or 1,
and/or a stereoisomeric form of the compound of the formula (I) and/or mixtures of this form in any ratio,
and/or a physiologically tolerated salt of the compound of the formula (I).

2. A compound of the formula (I) as claimed in claim 1, where
R⁴ is H, and
R¹, R², R³ and p are defined as in claim 1.

3. A compound of the formula (I) as claimed in claim 1, where
R⁴ is H, and
p is 0, and
R¹, R² and R³ are defined as in claim 1.

4. A compound of the formula (I) as claimed in claim 1, where
R² is H or methyl,
R⁴ is H,
p is 0, and
R¹ and R³ are defined as in claim 1.

5. A compound of the formula (I) as claimed in claim 1, where
R² is H or methyl,
R³ is a radical which is composed of from 1 to 2 amino acids and which can be N-terminally protected,
R⁴ is H,
p is 0, and
R¹ is defined as in claim 1.

6. A compound of the formula (I) as claimed in claim 1, where
R¹ is H,
R² is H or methyl,
R³ is a radical which is composed of from 1 to 2 amino acids and which can be N-terminally protected,
R⁴ is H, and
p is 0.

7. A compound of the formula (II) 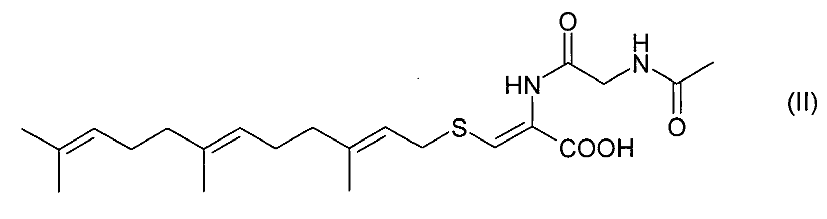 and the physiologically tolerated salts thereof.

8. Coniosulfide A, a compound which has the molecular formula C₂₂H₃₄O₄N₂S and is **characterized by** the ¹H NMR and ¹³C NMR data in accordance with Table 2, and physiologically tolerated salts of coniosulfide A.

9. A compound of the formula (III): 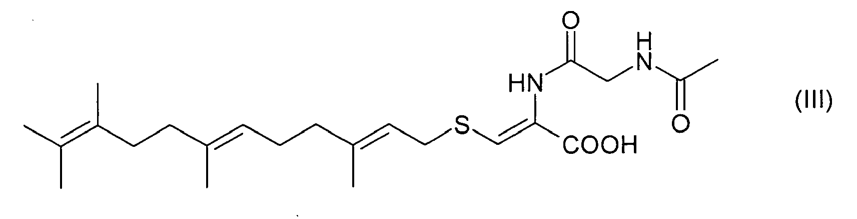 and the physiologically tolerated salts thereof.

10. Coniosulfide B, a compound which has the molecular formula C₂₃H₃₆O₄N₂S and is **characterized by** the ¹H NMR and ¹³C NMR data in accordance with Table 3, and physiologically tolerated salts of coniosulfide B.

11. A compound of the formula (IV) 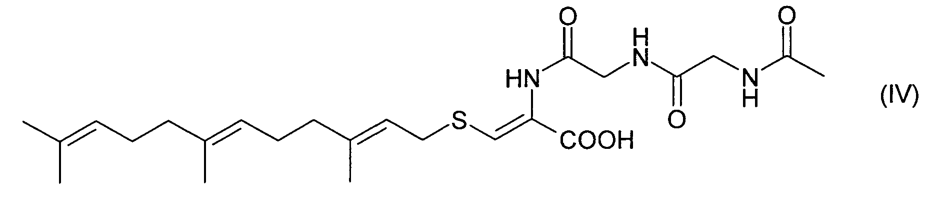 and the physiologically tolerated salts thereof.

12. Coniosulfide C, a compound which has the molecular formula C₂₄H₃₇O₅N₃S and is **characterized by** the ¹H NMR and ¹³C NMR data in accordance with Table 4, and physiologically tolerated salts of coniosulfide C.

13. A compound of the formula (V) 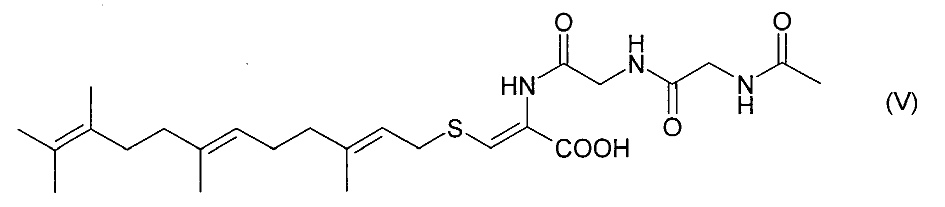 and the physiologically tolerated salts thereof.

14. Coniosulfide D, a compound which has the molecular formula C₂₅H₃₉O₅N₃S and is **characterized by** the ¹H NMR and ¹³C NMR data in accordance with Table 5, and physiologically tolerated salts of coniosulfide D.

15. A compound of the formula (I) as claimed in one of claims 1 to 6, which can be obtained by fermenting *Coniochaeta ellipsoidea* Udagawa, DSM 13856, or one of its mutants, in a culture medium until a compound of the formula (I) accumulates in the culture medium, and then isolating the compound of the formula (I), and optionally derivatizing it and/or, where appropriate, converting it into a pharmacologically tolerated salt.

16. A process for preparing a compound of the formula (I), which comprises fermenting the microorganism *Coniochaeta ellipsoidea* Udagawa, DSM 13856, or a variant or mutant of *Coniochaeta ellipsoidea* Udagawa, DSM 13856, in a culture medium until a compound of the formula (I) accumulates in the culture broth, isolating the compound of the formula (I) and then optionally derivatizing it and, where appropriate, converting it into a physiologically tolerated salt.

17. A pharmaceutical having a content of at least one compound as claimed in one or more of claims 1 to 15 and at least one physiologically suitable excipient and/or auxiliary substance.

18. A compound as claimed in one or more of claims 1 to 15 for use as a pharmaceutical.

19. A compound as claimed in one or more of claims 1 to 15 for the treatment and prophylaxis of Alzheimer's disease.

20. A process for producing a pharmaceutical as claimed in claim 17, which comprises bringing at least one compound as claimed in one or more of claims 1 to 15, together with at least one physiologically suitable excipient and/or auxiliary substance, into a suitable administration form.

## Revendications

1. Composé de formule (I) 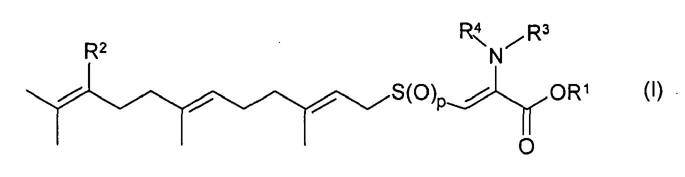 dans laquelle :
R¹
1.0 représente un atome d'hydrogène, ou
2.0 représente un radical choisi parmi un groupe -C₁-C₆-alkyle, un groupe -C₂-C₆-alcényle, un groupe -C₂-C₆-alcynyle ou un groupe -C₆-C₁₀-aryle, qui peut être mono-ou di-substitué par :
2.1 un groupe -OH,
2.2 un groupe =O,
2.3 un groupe -O-(C₁-C₆-alkyle) ,
2.4 un groupe -O-(C₂-C₆-alcényle),
2.5 un groupe -C₆-C₁₀-aryle,
2.6 un groupe -NH-C₁-C₆-alkyle,
2.7 un groupe -NH-C₂-C₆-alcényle,
2.8 un groupe -NH₂, ou
2.9 un atome de fluor, un atome de chlore, un atome de brome ou un atome d'iode,
où les substituants 2.3 à 2.8 peuvent également être davantage substitués par des fonctions -CN, -amide ou -oxime, ou
3.0 représente un groupe -(C₁-C₄-alkyl)-(C₆-C₁₀-aryle), ou
4.0 représente un groupe -NH₂, un groupe -NH-(C₁-C₆-alkyle), ou un groupe -NH-(C₂-C₆-alcényle),
R² représente un atome d'hydrogène, un groupe méthyle, un groupe éthyle, un groupe n-propyle, un groupe isopropyle, un groupe n-butyle ou un groupe isobutyle,
R³ représente un radical constitué de 1 à 10 acides aminés, qui peut être protégé sur un atome d'azote terminal,
R⁴ représente un atome d'hydrogène, un groupe -C₁-C₆-alkyle, un groupe -CO-(C₁-C₆-alkyle), et
p représente un entier 0, 1 ou 2,
à l'exception des composés de formule (I) dans lesquels simultanément :
R¹ représente un atome d'hydrogène,
R² représente un atome d'hydrogène,
R³ représente un groupe -CO-CH₃,
R⁴ représente un atome d'hydrogène, et
p vaut 0 ou 1,
et/ou une forme stéréoisomère du composé de formule (I) et/ou des mélanges de cette forme en un rapport quelconque,
et/ou un sel physiologiquement acceptable du composé de formule (I).

2. Composé de formule (I) selon la revendication 1, dans laquelle :
R⁴ représente un atome d'hydrogène, et
R¹, R², R³ et p sont tels que définis dans la revendication 1.

3. Composé de formule (I) selon la revendication 1, dans laquelle :
R⁴ représente un atome d'hydrogène, et
p vaut 0, et
R¹, R² et R³ sont tels que définis dans la revendication 1.

4. Composé de formule (I) selon la revendication 1, dans laquelle :
R² représente un atome d'hydrogène ou un groupe méthyle,
R⁴ représente un atome d'hydrogène,
p vaut 0, et
R¹ et R³ sont tels que définis dans la revendication 1.

5. Composé de formule (I) selon la revendication 1, dans laquelle :
R² représente un atome d'hydrogène ou un groupe méthyle,
R³ représente un radical constitué de 1 à 2 acides aminés, qui peut être protégé sur un atome d'azote terminal,
R⁴ représente un atome d'hydrogène,
p vaut 0, et
R¹ est tel que défini dans la revendication 1.

6. Composé de formule (I) selon la revendication 1, dans laquelle :
R¹ représente un atome d'hydrogène,
R² représente un atome d'hydrogène ou un groupe méthyle,
R³ représente un radical constitué de 1 à 2 acides aminés, qui peut être protégé sur un atome d'azote terminal,
R⁴ représente un atome d'hydrogène, et
p vaut 0.

7. Composé de formule (II) 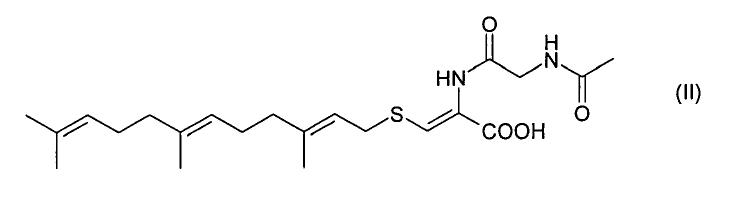 ainsi que des sels physiologiquement acceptables de celui-ci.

8. Coniosulfure A, un composé de formule moléculaire C₂₂H₃₄O₄N₂S et **caractérisé par** les données de RMN ¹H et ¹³C correspondant au Tableau 2, ainsi que des sels physiologiquement acceptables du coniosulfure A.

9. Composé de formule (III) 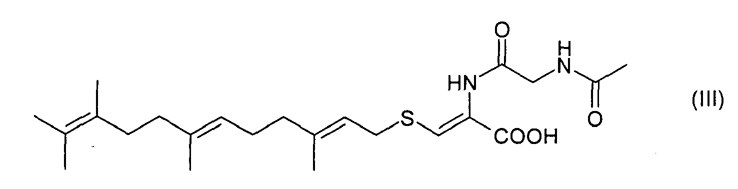 ainsi que des sels physiologiquement acceptables de celui-ci.

10. Coniosulfure B, un composé de formule moléculaire C₂₃H₃₆O₄N₂S et **caractérisé par** les données de RMN ¹H et ¹³C correspondant au Tableau 3, ainsi que des sels physiologiquement acceptables du coniosulfure B.

11. Composé de formule (IV) 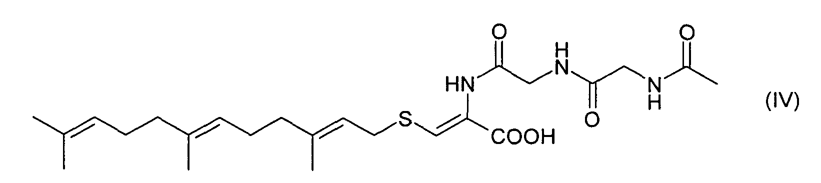 ainsi que des sels physiologiquement acceptables de celui-ci.

12. Coniosulfure C, un composé de formule moléculaire C₂₄H₃₇O₅N₃S et **caractérisé par** les données de RMN ¹H et ¹³C correspondant au Tableau 4, ainsi que des sels physiologiquement acceptables du coniosulfure C.

13. Composé de formule (V) 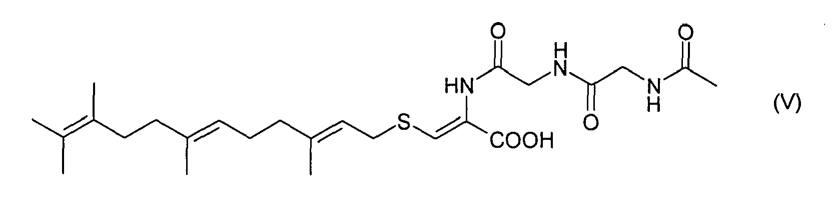 ainsi que des sels physiologiquement acceptables de celui-ci.

14. Coniosulfure D, un composé de formule moléculaire C₂₅H₃₉O₅N₃S et **caractérisé par** les données de RMN ¹H et ¹³C correspondant au Tableau 5, ainsi que des sels physiologiquement acceptables du coniosulfure D.

15. Composé de formule (I) selon l'une quelconque des revendications 1 à 6, pouvant être obtenu par la fermentation de *Coniochaeta ellipsoidea* Udagawa, DSM 13856, ou de l'un de ses mutants, dans un milieu de culture jusqu'à ce qu'un composé de formule (I) s'accumule dans le milieu de culture, et par l'isolation subséquente du composé de formule (I) et, éventuellement, la dérivatisation et/ou, le cas échéant, la transformation en un sel pharmacologiquement acceptable.

16. Procédé de préparation d'un composé de formule (I), **caractérisé en ce que** le microorganisme *Coniochaeta ellipsoidea* Udagawa, DSM 13856, ou une variante ou un mutant de *Coniochaeta ellipsoidea* Udagawa, DSM 13856, est fermenté dans un milieu de culture jusqu'à ce qu'un composé de formule (I) s'accumule dans le bouillon de culture, le composé de formule (I) est isolé et ensuite, éventuellement, dérivatisé et, le cas échéant, est transformé en un sel physiologiquement acceptable.

17. Médicament présentant une teneur en au moins un composé selon l'une ou plusieurs des revendications 1 à 15 et au moins un support et/ou un auxiliaire physiologiquement appropriés.

18. Composé selon l'une ou plusieurs des revendications 1 à 15, destiné à être utilisé en tant que médicament.

19. Composé selon l'une ou plusieurs des revendications 1 à 15, destiné au traitement et à la prophylaxie de la maladie d'Alzheimer.

20. Procédé de préparation d'un médicament selon la revendication 17, **caractérisé en ce qu'**au moins un composé selon l'une ou plusieurs des revendications 1 à 15 est mis sous une forme d'administration appropriée avec au moins un support et/ou un auxiliaire physiologiquement appropriés.
